# FASCICULE DE BREVET EUROPEEN

(11) **EP 1 625 400 B1**
(45) Date de publication et mention de la délivrance du brevet: **14.02.2007**
(21) Numéro de dépôt: 04742696.0
(22) Date de dépôt: 11.05.2004
(51) Int. Cl.: G01N 33/566

(54) **PROCEDE DE SELECTION DE REGULATEURS ALLOSTERIQUES DES RECEPTEURS COUPLES AUX PROTEINES G DE CLASSE III**
VERFAHREN ZUR SELEKTION ALLOSTERISCHER MODULATOREN VON G-PROTEIN GEKOPPELTEN REZEPTOREN DER FAMILIE III
METHOD FOR SELECTING ALLOSTERIC REGULATORS OF CLASS III G PROTEIN-COUPLED RECEPTORS

(30) Priorité: 22.05.2003 FR 0306136
(43) Date de publication de la demande: 15.02.2006
(73) Titulaire: CENTRE NATIONAL DE LA RECHERCHE SCIENTIFIQUE (CNRS), 75016 Paris (FR)
(72) Inventeur: GAVEN, Florence, F-34270 Saint-Mathieu De Trevier (FR); GOUDET, Cyril, F-34090 Montpellier (FR); PIN, Jean-Philippe, F-34000 Montpellier (FR); PREZEAU, Laurent, F-34170 Castelnau Le Lez (FR)
(74) Mandataire: Ahner, Francis
(86) Numéro de dépôt international: PCT/FR2004/001140
(87) Numéro de publication internationale: WO 2004/106932

(56) Documents cités:
- RAY KAUSIK ET AL: "Evidence for distinct cation and calcimimetic compound (NPS 568) recognition domains in the transmembrane regions of the human Ca2+ receptor" JOURNAL OF BIOLOGICAL CHEMISTRY, vol. 277, no. 21, 24 mai 2002 (2002-05-24), pages 18908-18913, XP002269528 ISSN: 0021-9258 cité dans la demande
- KNOFLACH FREDERIC ET AL: "Positive allosteric modulators of metabotropic glutamate 1 receptor: Characterization, mechanism of action, and binding site" PROCEEDINGS OF THE NATIONAL ACADEMY OF SCIENCES OF THE UNITED STATES, vol. 98, no. 23, 2001, pages 13402-13407, XP002269529 Nobember 6, 2001 ISSN: 0027-8424
- URWYLER S ET AL: "POSITIVE ALLOSTERIC MODULATION OF NATIVE AND RECOMBINANT GAMMA-AMINOBUTYRIC ACIDB RECEPTORS BY 2,6-DI-TERT-BUTYL-4-(3-HYDROXY- 2,2-DIMETHYL-PROPYL)-PHENOL (CGP7930) AND ITS ALDEHYDE ANALOG CGP13501" MOLECULAR PHARMACOLOGY, BALTIMORE, MD, US, vol. 60, no. 5, novembre 2001 (2001-11), pages 963-971, XP001153169 ISSN: 0026-895X
- GASPARINI FABRIZIO ET AL: "Allosteric modulators of group I metabotropic glutamate receptors: Novel subtype-selective ligands and therapeutic perspectives" CURRENT OPINION IN PHARMACOLOGY, vol. 2, no. 1, février 2002 (2002-02), pages 43-49, XP002269530 ISSN: 1471-4892
- REES S ET AL: "GPCR DRUG DISCOVERY THROUGH THE EXPLOITATION OF ALLOSTERIC DRUG BINDING SITES" RECEPTORS AND CHANNELS, HARWOOD ACADEMIC PUBLISHERS, CH, vol. 8, no. 5/6, 2002, pages 261-268, XP008017101 ISSN: 1060-6823
- KINGSTON A E ET AL: "LY341495 is a nanomolar potent and selective antagonist of group II metabotropic glutamate receptors" NEUROPHARMACOLOGY, vol. 37, no. 1, 1998, pages 1-12, XP002269531 ISSN: 0028-3908 cité dans la demande
- RAY ET AL.: "Calindol, a positive allosteric modulator of the human Ca2+ receptor, activates an extracellular ligand-binding domain deleted rhodopsin-like seven-transmembrane structure in the absence of Ca2+." JBC (PAPERS IN PRESS), 31 août 2005 (2005-08-31), pages 1-9,

## Description

La présente invention se rapporte au domaine des récepteurs couplés aux protéines G de classe III (RCPG-III), qui sont impliqués dans de nombreux phénomènes biologiques, pour la plupart vitaux, chez les organismes vivants.

Plus précisément, l'invention s'inscrit dans le cadre de la mise au point de moyens efficaces destinés à l'identification et la sélection de régulateurs allostériques des RCPG-III.

A cet effet, la présente invention concerne un procédé d'identification et sélection d'un composé agissant comme régulateur allostérique d'un RCPG-III selon la revendication 1. Selon ce procédé, le niveau d'activité d'un RCPG-III recombinant (RCPG-III-Δ), essentiellement dépourvu du domaine extracellulaire du RCPG-III natif correspondant, en présence d'un composé donné, est comparé à celui du même RCPG-III-Δ, en l'absence du composé en question. Le procédé est caractérisé en ce qu'il est mis en oeuvre en l'absence d'agoniste du RCPG-III, en ce que ledit RCPG-III est choisi dans le groupe comprenant :
- les récepteurs activés par le glutamate ;
- les récepteurs activés par le GABA ;
- les récepteurs putatifs de phéromones ; et
- les récepteurs du goût ;
et en ce que ledit régulateur allostérique se comporte comme un agoniste ou un agoniste inverse dudit RCPG-III-Δ.

Ce procédé permet ainsi d'identifier et sélectionner des activateurs ou inhibiteurs des RCPG-III.

L'invention vise en outre l'application d'un tel procédé à l'identification et la sélection de composés d'intérêt dans des domaines d'activités aussi variés que la pharmacie, l'industrie agro-alimentaire, l'environnement, l'écologie et la recherche fondamentale.

La comparaison des séquences des domaines heptahélices des RCPG a permis de répartir ces derniers dans trois grandes classes (Bockaert et Pin, 1999).

La classe I comprend les récepteurs semblables à la rhodopsine, tels que les récepteurs des catécholamines, des hormones glycoprotéiques et peptidiques.

La classe II rassemble non seulement les récepteurs de larges peptides tels que le glucagon et la sécrétine, mais également d'autres récepteurs tels que Frizzled, impliqués dans le développement.

La classe III des RCPG, quant à elle, comprend les récepteurs activés par les deux principaux neurotransmetteurs, à savoir le glutamate et l'acide γ-aminobutyrique (GABA), les récepteurs senseurs des ions calcium, les récepteurs putatifs de phéromones, ainsi que les récepteurs responsables de la détection des goûts sucrés et "umami" (acides aminés, en particulier le glutamate).

La présente invention concerne plus particulièrement les RCPG-III de la revendication 1.

Huit gènes codant pour des RCPG-III activés par le glutamate (également appelés récepteurs métabotropiques du glutamate ou mGlu), ont été identifiés chez les mammifères (Conn et Pin, 1997; Pin *et al.,* 2003). Ces mGlu peuvent être classés en trois groupes sur la base de leur pharmacologie, leur similarité de séquence et leur transduction.

Dans le groupe I, les récepteurs mGlu1 et mGlu5 activent la phospholipase C (PLC) et semblent agir en synergie avec les récepteurs canaux du glutamate. Ils apparaissent souvent comme des agents qui facilitent la communication glutamatergique.

Ainsi, les régulateurs des mGlu du groupe 1 semblent posséder des propriétés favorisant la mémoire, utiles pour le traitement de maladies telles que la maladie d'Alzheimer ou la schizophrénie.

Les récepteurs du groupe II, mGlu2 et mGlu3, de même que les récepteurs du groupe III, mGlu4, mGlu6, mGlu7 et mGlu8, sont couplés négativement à l'adénylyl cyclase. Ils régulent également l'activité de canaux calciques et potassiques. Ils sont souvent localisés en pré-synapse, d'où ils inhibent la libération de glutamate.

De fait, des agonistes ou des régulateurs allostériques positifs des mGlu des groupes II ou III présentent des propriétés anxiolytiques, anti-épileptiques, antipsychotiques, anti-parkinsonniennes, et peuvent diminuer les effets de dépendance des drogues telles que le tabac et la cocaïne (Conn et Pin, 1997).

Un seul gène codant un récepteur activé par les ions calcium a été identifié (Brown et MacLeod, 2001). Ce récepteur joue un rôle clef dans la régulation de la calcémie en contrôlant la libération de parathormone, l'absorption de calcium par les os, ainsi que l'excrétion du calcium au niveau du rein. Il aurait également un rôle au niveau central. Des mutations de ce gène sont à l'origine de maladies telles que l'hypocalciurie familiale, l'hyperparathyroidisme néonatal sévère (mutations entraînant une perte de fonction) (Pollak *et al.,* 1993), ou l'hypocalcémie autosomale dominante (mutations entraînant un gain de fonction) (Pollak *et al.,* 1994). D'autre part, l'inhibition de ces récepteurs permettrait de corriger certaines maladies telle que l'ostéoporose.

A ce jour, seuls deux gènes codant des récepteurs GABA_{B}, GB1 et GB2, ont été identifiés. Cependant, ni GB1 ni GB2 ne sont capables, lorsqu'ils sont pris isolément, de former un récepteur GABA_{B} fonctionnel. L'association des deux protéines est en effet nécessaire à la formation d'un récepteur au propriétés proches du récepteur endogène (Marshall *et al.*, 1999).

Le récepteur GABA_{B} a été le premier RCPG découvert comme ne fonctionnant que sous forme hétérodimérique. Il a ainsi été montré que le GABA ne se fixe que sur GB1 (Kniazeff *et al.,* 2002), et que GB2 est nécessaire à l'adressage de GB1 à la surface (Pagano *et al.,* 2001). GB2 est également nécessaire à la haute affinité des agonistes sur GB1 (Kaupmann *et al.,* 1998). En outre, GB2 est crucial pour le couplage aux protéines G (Duthey *et al.,* 2002).

Le récepteur GABA_{B} constitue donc un excellent modèle d'étude du rôle de la dimérisation des RCPG dans leur mécanisme d'activation.

De plus, des agents régulateurs de ce récepteur permettraient de développer de nouveaux traitements contre l'épilepsie et certaines formes de douleur.

Il a également été montré que les récepteurs du goût T1R1, T1R2 et T1R3 fonctionnaient sous forme hétérodimérique (Nelson *et al.,* 2002). Ces récepteurs sont responsables de la détection des molécules sucrées, parmi lesquels les édulcorants tels que l'aspartame. La découverte d'agents régulateurs de ces récepteurs revêt un intérêt dans le domaine de l'industrie agro-alimentaire.

Conformément à l'acception usuelle, l'on distingue les ligands dits « orthostériques », se fixant au niveau du site actif des RCPG-III, des régulateurs « allostériques », capables de se lier aux RCPG-III au niveau d'un site autre que le site actif. En l'occurrence, le site actif des RCPG-III est porté par le domaine extracellulaire. Des régulateurs allostériques se fixent sur le domaine membranaire des RCPG-III.

De manière classique, un « agoniste » est une molécule capable, à elle seule, d'augmenter l'activité d'un récepteur.

Un « antagoniste » est une molécule capable d'inhiber l'effet activateur d'un agoniste. Parmi les antagonistes, l'on distingue les « antagonistes compétitifs », qui agissent au niveau du site orthostérique, des « antagonistes non compétitifs », qui agissent au niveau d'un site allostérique.

Un « agoniste inverse » est une molécule capable d'inhiber l'activité constitutive d'un récepteur, i.e., l'activité mesurable en l'absence de tout agoniste), lorsqu'une telle activité est effectivement mesurable. Un agoniste inverse est en outre capable d'inhiber l'effet d'un agoniste. Il s'agit donc également d'un antagoniste.

Un « régulateur allostérique positif » est une molécule capable de faciliter l'action d'un agoniste. Un tel régulateur agit sur un site différent du site orthostérique, au niveau duquel se fixe le ligand naturel.

L'on entend ici par « faciliter l'action d'un agoniste », soit augmenter la puissance dudit agoniste (de plus faibles concentrations d'agonistes seront alors suffisantes pour produire le même effet), soit augmenter son efficacité (i.e., augmenter la réponse maximale obtenue avec une concentration saturante d'agoniste, c'est à dire une concentration suffisante pour occuper tous les récepteurs).

Un « régulateur allostérique négatif » est une molécule capable de diminuer l'effet d'un agoniste, et ce, en agissant sur un site autre que le site orthostérique. Un tel régulateur est également appelé « antagoniste non-compétitif ». Comme dans le cas des antagonistes, un régulateur allostérique négatif peut ou non posséder une activité agoniste inverse.

Au sens de l'invention, les expressions « inhiber l'effet d'un agoniste » et « diminuer l'effet d'un agoniste » sont équivalentes.

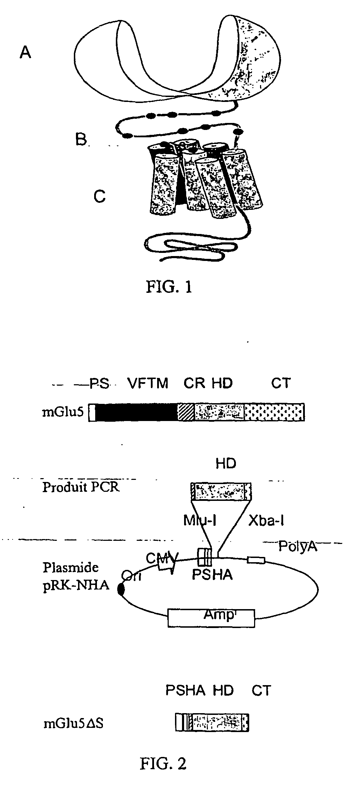

Tous les RCPG-III connus à ce jour ont la particularité d'être constitués de deux domaines protéiques distincts : un domaine extracellulaire appelé « Venus flytrap », responsable de la liaison des ligands naturels (ligands orthostériques), et un domaine membranaire heptahélice responsable du couplage aux protéines G et cible des régulateurs allostériques (Pin *et al.,* 2003) (Figure 1). Ces deux domaines sont séparés par une région riche en cystéines.

Une deuxième caractéristique spécifique des RCPG-III réside dans leur capacité à former des dimères constitutifs, souvent reliés entre eux par au moins un pont disulfure.

La construction d'un récepteur calcium recombinant, tronqué de son domaine extracellulaire, a été décrite par Ray et Northup (2002). Les auteurs ont recherché, à l'aide de composés connus pour agir comme régulateurs allostériques du récepteur Ca²⁺ humain, si le domaine transmembranaire de ce récepteur comprenait ou non des déterminants structuraux responsables de la liaison de ligands et de l'activation subséquente dudit récepteur. Les auteurs ont ainsi démontré que, à l'instar du domaine extracellulaire, le domaine transmembranaire heptahélice contient un ou plusieurs sites de liaison des ions calcium. Cependant, l'activation du récepteur recombinant par les ions calcium n'a été détectée qu'en présence d'un autre ligand.

Les régulateurs allostériques positifs ou négatifs des RCPG-III jusque-là décrits ont été identifiés par des techniques de criblage à haut débit.

Les "calcimimétiques" ont été mis en évidence pour leur capacité à réguler l'activité du récepteur calcium (Nemeth *et al.*, 1998; Hammerland *et al.,* 1998; Hammerland *et al.,* 1999)

Des régulateurs positifs du récepteur GABA_{B} (Urwyler, *et al.,* 2001), du récepteur mGlu1 (Knoflach *et al.,* 2001), mais aussi des récepteurs mGlu5, mGlu2 et mGlu4 ont été présentés lors du congrès mGlu2002 (Taormina, Sicile, Italie, septembre 2002).

Selon les techniques de criblage à haut débit utilisées jusqu'à présent, des chimiothèques sont criblées grâce à des tests fonctionnels réalisés au moyen de-lignées cellulaires exprimant un RCPG-III de façon stable. Les composés présents dans la chimiothèque sont testés pour leur capacité à potentialiser l'effet d'une concentration donnée d'agoniste.

Cependant, une telle approche se heurte à des difficultés multiples.

En premier lieu, il est nécessaire d'obtenir des lignées cellulaires exprimant ces récepteurs de manière stable. Or, il s'agit là d'une étape délicate, surtout dans le cas des récepteurs du glutamate, dans la mesure où cet acide aminé est présent dans tous les milieux de culture, ainsi que dans le sérum. Il est de surcroît produit par la plupart des lignées cellulaires. En conséquence, il s'avère en pratique difficile, voire impossible, d'abaisser la concentration du glutamate dans le milieu de culture à un niveau suffisamment faible pour que les récepteurs ne soient pas activés par le glutamate environnant.

Pour remédier à ce problème, le groupe Eli Lilly & Co a développé une lignée cellulaire qui exprime un transporteur à haute affinité du glutamate (cellules RGT établies sur la base d'une lignée AV12) (Kingston *et al.,* 1998; Schoepp *et al.,* 1997). Les cellules ainsi mises au point contrôlent la concentration extracellulaire du glutamate. Elles maintiennent cette concentration à des valeurs suffisamment faibles pour que les récepteurs exprimés ne soient pas activés en permanence par le glutamate présent dans le milieu. Ceci facilite l'établissement de lignées exprimant les récepteurs de façon stable. Cependant, un tel système présente des inconvénients. En effet, parmi les composés testés, certains peuvent être pris en charge par le transporteur du glutamate et transportés dans les cellules, ce qui peut abaisser leur concentration et prévenir leur action normale sur les récepteurs. En outre, la capacité de certaines des molécules testées à inhiber le transporteur du glutamate peut entraîner des difficultés dans la mise en oeuvre du test de criblage. En pareil cas, la concentration en glutamate croît, ce qui se traduit par une augmentation artéfactuelle de l'activité du récepteur. Le composé apparaît alors comme un régulateur allostérique positif du récepteur, et ce, même s'il n'en est pas véritablement un. Un crible secondaire est donc nécessaire.

En second lieu, les régulateurs allostériques n'ont bien souvent aucun effet sur le RCPG-III si celui-ci n'est pas simultanément activé par de faibles concentrations d'agoniste. La mise au point d'un test de sélection reproductible et fiable est alors rendue plus ardue par l'addition nécessaire d'agoniste. Se pose en effet la question de savoir à quelle concentration utiliser l'agoniste. Si cette concentration n'est pas suffisante ou, au contraire, est trop élevée, l'effet du régulateur allostérique ne sera probablement pas détectable. En outre, le rapport signal sur bruit de la réponse du régulateur allostérique se trouve réduit du fait de l'augmentation de la réponse de base due à la présence de l'agoniste.

La présente invention permet précisément de contourner l'ensemble des problèmes mentionnés ci-dessus, via l'utilisation de récepteurs recombinants dépourvus de leur domaine de liaison extracellulaire.

En effet, l'invention démontre pour la première fois que le domaine membranaire d'un RCPG-III est suffisant pour fixer des régulateurs allostériques, et ce, avec une affinité comparable à celle observée en utilisant le récepteur sauvage correspondant.

Ainsi, ces récepteurs recombinants sont insensibles au glutamate.

Avantageusement, dans le cadre de la présente invention, les régulateurs allostériques se comportent de manière tout à fait surprenante comme de véritables agonistes ou antagonistes des récepteurs recombinants.

Cette dernière caractéristique confère à la présente invention un avantage considérable, dans la mesure où il est en pratique plus aisé d'étudier l'effet agoniste d'une molécule sur un récepteur tronqué, que l'effet régulateur allostérique positif de ladite molécule sur le récepteur sauvage correspondant.

Ainsi, avantageusement, de tels récepteurs recombinants peuvent donc être exprimés de manière stable par les cellules. En outre, la présence additionnelle d'agoniste s'avère inutile. Enfin, le procédé objet de l'invention utilisant ces récepteurs recombinants permet d'obtenir un meilleur rapport signal sur bruit.

La présente invention a pour objet un procédé d'identification et sélection d'un composé agissant comme régulateur allostérique d'un RCPG-III choisi parmi :
- les récepteurs activés par le glutamate ;
- les récepteurs activés par le GABA ;
- les récepteurs putatifs de phéromones ; et
- les récepteurs du goût.

Dans le cadre de l'invention, un « composé » est défini comme étant n'importe quel type de molécule, biologique ou chimique, naturelle, recombinante ou synthétique. Par exemple, un composé peut être un acide nucléique (e.g., un oligonucléotide), une protéine, un acide gras, un anticorps, un polysaccharide, un stéroïde, une purine, une pyrimidine, une molécule organique, un radical chimique, etc... Le terme « composé » couvre également les fragments, dérivés, analogues structurels ou combinaisons de ceux-ci, dès lors qu'ils agissent comme régulateurs allostériques d'un RCPG-III.

La capacité à agir comme des régulateurs allostériques d'un RCPG-III représente la propriété d'intérêt des composés identifiés et sélectionnés par la mise en oeuvre du procédé objet de la présente invention.

Ce procédé, qui est caractérisé en ce qu'il est mis en oeuvre en l'absence d'agoniste du RCPG-III, en ce que ledit RCPG-III est choisi dans le groupe comprenant :
- les récepteurs activés par le glutamate ;
- les récepteurs activés par le GABA ;
- les récepteurs putatifs de phéromones ; et
- les récepteurs du goût ;
et en ce que ledit régulateur allostérique se comporte comme un agoniste ou un agoniste inverse dudit RCPG-III-Δ, comprend au moins les étapes suivantes :
a) la production d'un RCPG-III recombinant (RCPG-III-Δ) essentiellement dépourvu du domaine extracellulaire du RCPG-III natif correspondant ;
b) la mise en présence dudit composé avec ledit RCPG-III-Δ ;
c) la mesure du niveau N₁ d'activité dudit RCPG-III-Δ en présence dudit composé ;
d) la comparaison du niveau N₁ mesuré à l'étape c) avec le niveau N₀ d'activité du RCPG-III-Δ en l'absence dudit composé ; et
e) si N₁ est significativement différent de N_{O,} l'identification et la sélection dudit régulateur allostérique.

Avantageusement, ledit RCPG-III-Δ est totalement dépourvu du domaine extracellulaire dudit RCPG-III natif.

La mise en oeuvre du procédé susmentionné permet d'identifier et sélectionner des composés agissant comme des régulateurs allostériques d'un RCPG-III, en se fixant à ce dernier au niveau de son domaine héptahélice.

Selon un premier mode de mise en oeuvre du procédé objet de l'invention, si N₁ est significativement supérieur à N₀, ledit composé se comporte comme un agoniste dudit RCPG-III-Δ, et est un régulateur allostérique positif du récepteur entier.

Selon un deuxième mode de mise en oeuvre du procédé objet de l'invention, si N₁ est significativement inférieur à N₀, ledit composé se comporte comme un agoniste inverse dudit RCPG-III-Δ, et est un régulateur allostérique négatif du récepteur entier.

Dans le contexte de l'invention, la mesure du niveau d'activité du RCPG-III-Δ peut être effectuée par n'importe quelle méthode conventionnelle, connue de l'homme du métier, adaptée à la mesure de l'activité d'un RCPG-III. Parmi ces méthodes, l'on peut citer, à titre d'exemple, la mesure du niveau de production d'inositols phosphates (IP ; voir Partie Expérimentale, infra ; Berridge, 1983 ; Brandish *et al.*, 2003), la mesure de la liaison des GTPγ (Milligan, 2003), la mesure des signaux calciques intracellulaires à l'aide de marqueurs fluorescents tels que le Fluo3 ou le Fluo4, et la mesure de la production d'AMPc, ces deux dernières méthodes étant classiques et bien connues de l'homme du métier.

Le procédé conforme à l'invention peut être mis en oeuvre avec un RCPG-III-Δ comprenant :
- un domaine membranaire heptahélice, sans signal d'adressage ; ou
- un signal d'adressage et un domaine membranaire heptahélice.

Au sens de la présente invention, l'on entend par « signal d'adressage », une séquence d'acides aminés, de longueur variable, en général de 15 à 35 résidus environ, permettant l'insertion correcte d'un récepteur dans la membrane plasmique. En particulier, un tel signal d'adressage peut être :
- un peptide signal conforme à l'acception usuelle ; ou
- les 20 premiers acides aminés de l'extrémité N-terminale de la rhodopsine, notamment d'origine bovine (Ray et Northup, 2002).

Dans le cas où ledit signal d'adressage est un peptide signal, ce peptide signal peut être celui du RCPG-III natif correspondant, ou celui d'un RCPG-III sauvage différent, ou encore n'importe quel peptide signal connu. De préférence, ledit peptide signal est celui dudit RCPG-III natif correspondant.

Avantageusement, l'étape a) du procédé susmentionné comprend au moins les sous-étapes suivantes :
a1) l'amplification par PCR d'un acide nucléique codant ledit domaine membranaire dudit RCPG-III-Δ ;
a2) le clonage du produit d'amplification dans un vecteur d'expression recombinant ;
a3) la transfection dudit vecteur recombinant dans une cellule hôte recombinante ; et
a4) la production dudit RCPG-III-Δ par ladite cellule hôte recombinante.

De manière particulière, dans le cas où un signal d'adressage est utilisé, la sous-étape a2) consiste à cloner, dans un vecteur d'expression recombinant, le produit d'amplification en aval d'un acide nucléique codant ledit signal d'adressage.

Dans le cas où le RCPG-III en cause possède, dans sa partie intracellulaire, un signal responsable de sa rétention dans le réticulum endoplasmique (Pagano *et al.*, 2001), il convient au préalable de rendre ce signal non fonctionnel par mutation, selon des méthodes de mutagénèse conventionnelles, connues de l'homme du métier, afin de permettre un adressage du RCPG-III à la membrane plasmique.

Il est avantageux de placer l'expression de l'acide nucléique codant le domaine membranaire du RCPG-III-Δ, ou la co-expression des acides nucléiques codant le signal d'adressage et le domaine membranaire du RCPG-III-Δ, sous le contrôle d'un promoteur fort porté par le vecteur recombinant.

A cet effet, l'homme du métier pourra utiliser n'importe quel promoteur transcriptionnel fort connu. Par exemple, un tel promoteur fort peut être le promoteur du cytomégalovirus (CMV ; voir Figure 2) ou celui du virus SV40.

De multiples vecteurs sont à la disposition de l'homme du métier pour construire le récepteur RCPG-III-Δ. Des vecteurs conventionnels appropriés sont, en particulier, les vecteurs pRK5-NHA (voir Partie Expérimentale infra), pcDNA3 (InVitrogene) et pCl (Promega).

En outre, aux fins de la mise en oeuvre du procédé selon l'invention, l'homme du métier pourra utiliser, à titre de cellule hôte recombinante, n'importe quel type de cellule permettant l'expression hétérologue d'une protéine. L'homme du métier pourra notamment choisir parmi les cellules eucaryotes, telles que les cellules de levure, les cellules HEK293 et COS, et les cellules procaryotes, telles les bactéries.

En particulier, ledit RCPG-III est un récepteur activé par le glutamate, et de préférence, le récepteur mGlu1, mGlu2 ou mGlu5.

Alternativement, ledit RCPG-III est un récepteur activé par la GABA, et de préférence, la sous-unité GB2.

Selon un deuxième aspect, la présente invention concerne les applications du procédé tel que décrit ci-dessus, dans divers domaines, notamment dans les domaines de la pharmacie, l'écologie et l'environnement, l'industrie agro-alimentaire, la recherche, etc...

Selon un premier mode de réalisation, l'invention vise l'application du procédé défini supra à l'identification et la sélection d'un composé utile dans le cadre d'un traitement prophylactique et/ou thérapeutique chez les mammifères, y compris l'homme.

De manière particulière, un tel composé peut convenir à la mise en oeuvre d'un traitement concernant au moins une maladie parmi :
- la maladie d'Alzheimer, la schizophrénie, les troubles nerveux, la dépression, l'épilepsie, la psychose, la dépendance aux drogues (RCPG-III activés par le glutamate) ;
- l'épilepsie, la douleur (RCPG-III activés par le GABA).

Selon un deuxième mode de réalisation, l'invention a trait à l'application du procédé décrit précédemment à l'identification et la sélection d'un composé utile dans le cadre d'un traitement destiné à augmenter ou, au contraire, à réduire la prolifération d'organismes vivants, tels les insectes et les vers, dont le génome contient au moins un RCPG-III (Parmentier *et al.*, 1996 ; Pin *et al.*, 2003).

Selon un troisième mode de réalisation, l'invention concerne plus précisément les récepteurs du goût. A cet égard, la présente invention a pour objet l'application du procédé ci-dessus à l'identification et la sélection d'un composé utile comme additif alimentaire.

En particulier, un tel additif alimentaire est destiné à conférer un goût sucré, tel un édulcorant, ou un goût umami, ou augmente la sensibilité de détection d'un composé sucré.

Les figures suivantes sont fournies à titre purement illustratif et ne limitent en aucune façon l'objet de la présente invention.

Figure 1 : schéma de la structure générale d'un RCPG-III.
A : domaine externe dit "Venus flytrap", site de liaison des ligands orthostériques (ou agonistes) ;
B : région riche en cystéines ; et
C : domaine membranaire heptahélice, site de liaison des régulateurs allostériques.

Figure 2 : schéma de construction du plasmide d'expression du récepteur tronqué mGlu5ΔS.
PS : peptide signal ; VFTM : module « Venus flytrap » ; CR : région riche en cystéines ; HD : domaine membranaire heptahélice ; CT : domaine carboxy-terminal intracellulaire ; CMV : promoteur du cytomégalovirus ; HA : étiquette hémagglutinine.

Figure 3 : graphique représentant l'effet du glutamate sur les récepteurs mGlu5 et mGlu5ΔS, dans des cellules HEK293 ayant été transfectées transitoirement avec l'ADNc codant lesdits récepteurs.
Barres blanches production d'IP basale ;
Barres grises : production d'IP en présence de 100 µM de glutamate.
Les résultats correspondent au rapport de la production d'IP sur la radioactivité restant dans les membranes. Ces données correspondent à la moyenne ± écart type à la moyenne de triplicatas provenant d'une expérience représentative.

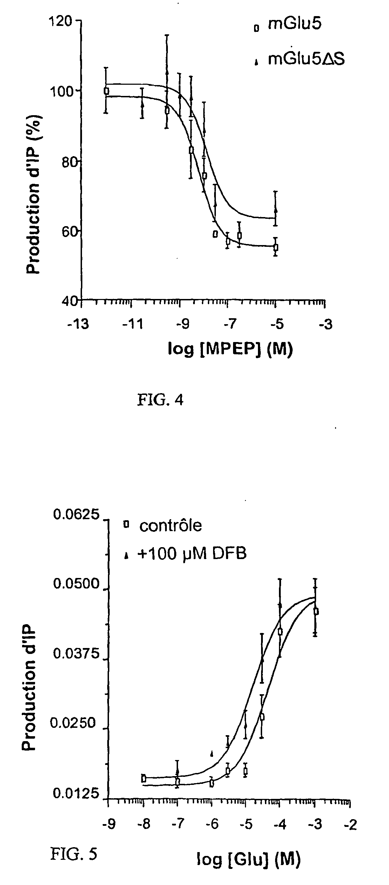

Figure 4 : graphique représentant l'effet dose du MPEP sur les récepteurs mGlu5 et mGlu5ΔS, dans des cellules HEK293 exprimant les récepteurs mGlu5 (carrés blancs) ou mGlu5ΔS (triangles noirs) de façon transitoire. Les résultats correspondent au rapport de la production d'IP sur la radioactivité restant dans les membranes et sont exprimés en pourcentage de la production basale d'IP. Ces données correspondent à la moyenne ± écart type à la moyenne de triplicatas provenant d'une expérience représentative.

Figure 5 : graphique représentant l'effet dose du glutamate sur le récepteur mGlu5 en présence (triangles noirs) ou en absence (carrés blancs) de DFB, dans des cellules HEK293 transfectées avec l'ADNc codant ledit récepteur.
Les résultats correspondent au rapport de la production d'IP sur la radioactivité restant dans les membranes. Ces données correspondent à la moyenne ± écart type à la moyenne de triplicatas provenant d'une expérience représentative.

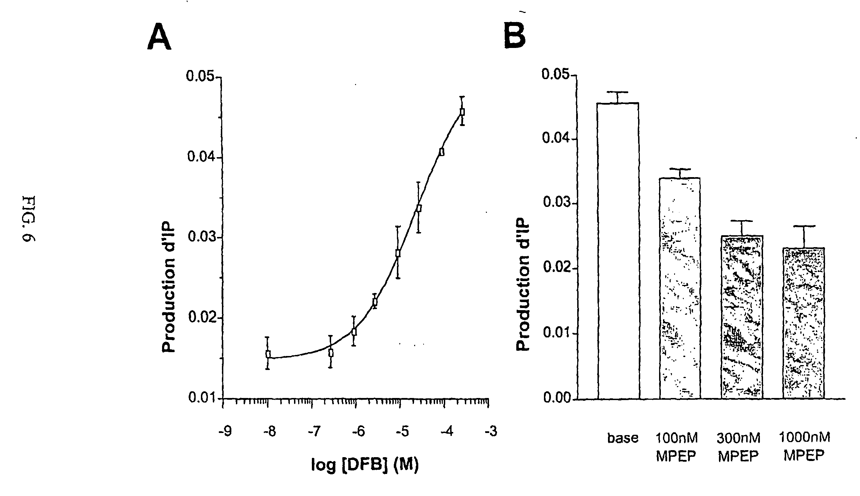

Figure 6 : graphiques représentant l'effet dose du DFB sur mGlu5ΔS et son inhibition par le MPEP.
A: effet de doses croissantes de DFB sur la production d'IP par des cellules HEK293 exprimant de manière transitoire le récepteur mGlu5ΔS.
B : effet de différentes concentrations de MPEP sur la production d'IP par des cellules exprimant le récepteur mGlu5ΔS traitées avec 300 µM de DFB.
Les résultats correspondent au rapport de la production d'IP sur la radioactivité restant dans les membranes. Ces données correspondent à la moyenne ± écart type à la moyenne de triplicatas provenant d'une expérience représentative.

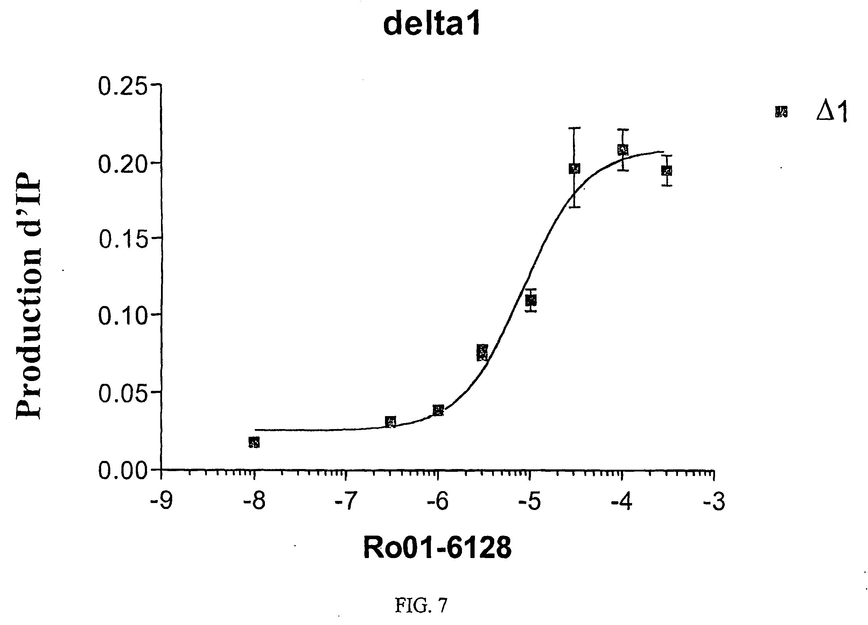

Figure 7: graphique représentant l'effet dose du Ro01-6128 sur le récepteur delta1s, dans les cellules HEK293 exprimant ce récepteur de façon transitoire. Les résultats correspondent au rapport de la production d'IP sur la radioactivité restant dans les membranes. Ces données correspondent à la moyenne ± écart type à la moyenne de triplicats provenant d'une expérience représentative.

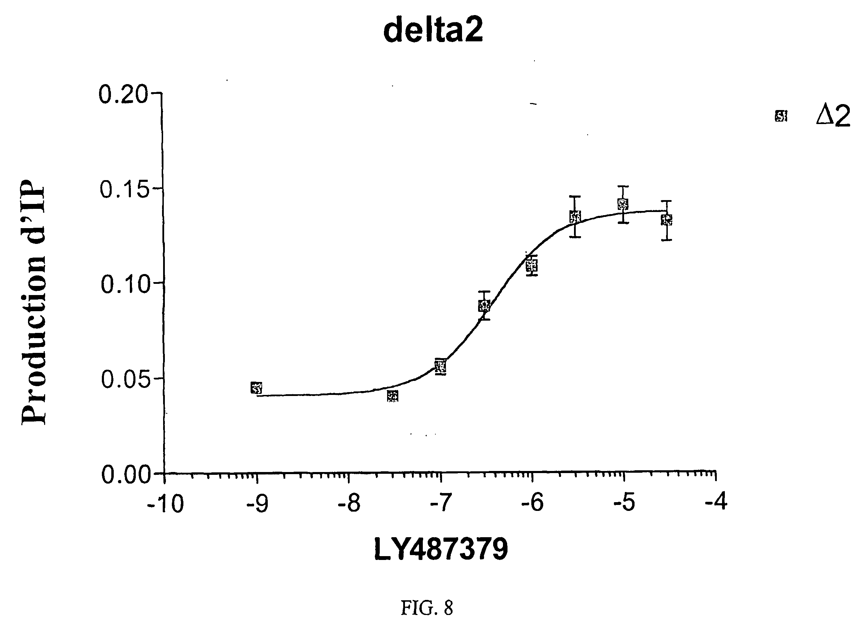

Figure 8: graphique représentant l'effet dose du LY487379 sur le récepteur delta2s, dans les cellules HEK293 exprimant ce récepteur de façon transitoire, ainsi que la protéine G chimérique Gqi9 permettant le couplage de ce récepteur à la voie d'activation de la phospholipase C. Les résultats correspondent au rapport de la production d'IP sur la radioactivité restant dans les membranes. Ces données correspondent à la moyenne ± écart type à la moyenne de triplicats provenant d'une expérience représentative.

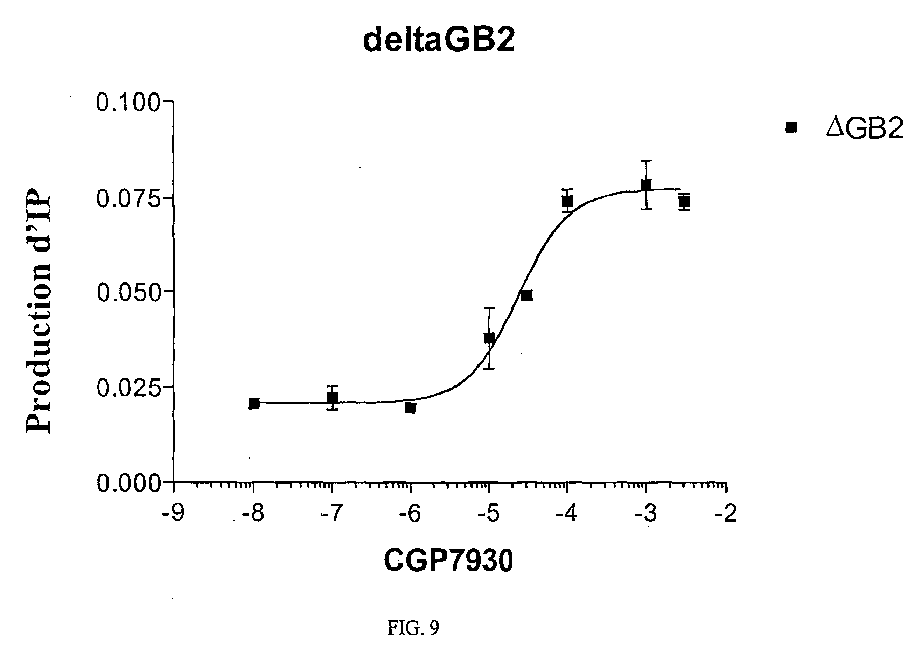

Figure 9: graphique représentant l'effet dose du CGP7930 sur le récepteur deltaGB2s, dans les cellules HEK293 exprimant ce récepteur de façon transitoire, ainsi que la protéine G chimérique Gqi9 permettant le couplage de ce récepteur à la voie d'activation de la phospholipase C. Les résultats correspondent au rapport de la production d'IP sur la radioactivité restant dans les membranes. Ces données correspondent à la moyenne ± écart type à la moyenne de triplicats provenant d'une expérience représentative

La partie expérimentale ci-après, appuyée par des exemples et des figures, illustre de manière non limitative l'invention.

### PARTIE EXPERIMENTALE

### A. Récepteur mGlu5

### I. Construction du plasmide codant un récepteur RCPG-III tronqué (protéine mGlu5ΔS)

La partie du cDNA du récepteur mGlu5 codant son domaine heptahélice entre le résidu P568, situé 11 résidus avant le premier domaine transmembranaire, et le résidu L864, situé 37 résidus après le 7^{ème} domaine transmembranaire, a été amplifiée par PCR (amorces SEQ ID N°1 et SEQ ID N°2) en incluant respectivement les sites de restriction des enzymes *Mlu-*I et *Xba*-I aux extrémités 5' et 3' (Figure 2). Le produit PCR ainsi obtenu a été digéré par les enzymes *Mlu-*I et *Xba*-I, sous-cloné dans le vecteur pRK5-NHA (Figure 2), et digéré par les mêmes enzymes. Le plasmide pRK5-NHA a été obtenu à partir du plasmide pRKG5a-NHA (Ango *et al.,* 1999), en remplaçant la séquence codant le récepteur mGlu5, de la fin de l'étiquette HA jusqu'au site de restriction *Xba*I, par un site de restriction *Mlu*I. Le plasmide obtenu code donc une protéine possédant le peptide signal du récepteur mGlu5, suivi de l'étiquette HA (PYDVPDYA, SEQ ID N°3), puis du domaine heptahélice du récepteur mGlu5 tronqué de la majorité de son domaine C-terminal intracellulaire. L'expression de cette protéine est placée sous le contrôle d'un promoteur fort CMV permettant son expression transitoire après transfection dans des lignées cellulaires HEK293 ou COS.

### II. Résultats

### II.1. Expression fonctionnelle de la protéine tronquée mGlu5ΔS :

Après transfection du plasmide construit conformément à la procédure décrite en partie I, dans les cellules HEK293, l'expression d'une protéine reconnue par un anticorps anti-HA a pu être vérifiée par western blot ainsi que par immunohistochimie (résultats non montrés). En outre, les cellules transfectées ont été marquées par l'anticorps-anti-HA même si elles n'étaient pas perméabilisées. Du fait que l'épitope HA est placé à l'extrémité N-terminale de la protéine mGlu5ΔS (voir Figure 2), et se situe donc au niveau extracellulaire, il a été prouvé par immunohistochimie que la protéine était correctement adressée au niveau de la membrane plasmique.

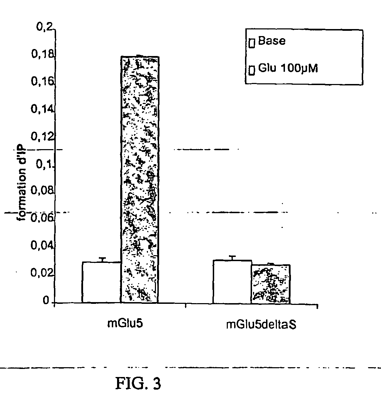

L'analyse du couplage du récepteur sauvage mGlu5 à la voie de production des inositols phosphates (IP) dans les cellules HEK293 a montré que ce récepteur était partiellement actif même en absence de glutamate (Joly *et al.,* 1995). Selon le modèle de fonctionnement des mGlus, il avait été proposé que cette activité constitutive du récepteur provenait d'un équilibre dynamique entre des états inactifs et des états actifs du domaine heptahélice du récepteur (Parmentier *et al.,* 2002). En accord avec cette hypothèse, l'expression de la protéine mGlu5ΔS dans les cellules HEK293 a conduit à une production basale d' IP plus importante que celle mesurée dans les cellules contrôles. Conformément au fait que le glutamate se fixe au niveau du domaine externe du récepteur, ce dernier stimulait la production d'IP dans les cellules exprimant le récepteur sauvage mGlu5, mais pas dans les cellules exprimant la protéine mGlu5ΔS (Figure 3).

### II.2. Effet des régulateurs allostériques sur la fonction de la protéine tronquée mGlu5ΔS :

La production basale d'IP dans les cellules exprimant le récepteur mGlu5 sauvage (témoin de l'activité constitutive du récepteur) pouvait être inhibée par le régulateur allostérique négatif MPEP (Pagano *et al.,* 2000) avec un Cl50 (concentration en régulateur allostérique négatif nécessaire pour obtenir 50% de son effet maximal) de 5,7 ± 0,6 µM (n=4) (Figure 4). Cet effet du MPEP était également observé sur les cellules exprimant la protéine mGlu5ΔS, avec un Cl50 de 9,8±2,5 µM (n=3) (Figure 4), non différent significativement de la valeur déterminée sur le récepteur sauvage. Ceci a confirmé que le MPEP agit au niveau du domaine heptahélice du récepteur, et que le domaine extracellulaire n'a pas d'influence sur son affinité apparente. Ce résultat a également démontré que- l'activité basale de production d'IP dans les cellules HEK293 exprimant la protéine mGlu5ΔS, résulte bien de la capacité de cette protéine à activer la voie intracellulaire de façon constitutive.

Le DFB a récemment été décrit comme un régulateur allostérique positif du récepteur mGlu5 (Williams *et al.,* 2002). Afin de tester son effet sur la protéine mGlu5ΔS, du DFB a été synthétisé par F. Acher (UMR Université R. Descartes, rue des Saints Pères, Paris 5). En accord avec les données publiées, le DFB n'activait pas de façon significative le récepteur mGlu5 sauvage dans des conditions où la concentration de glutamate dans le milieu d'incubation était maintenue au plus bas par la co-expression du transporteur du glutamate EAAC1, et par l'ajout de l'enzyme GPT (qui dégrade le glutamate en présence de pyruvate (2mM)) dans le milieu d'incubation (Figure 5). En revanche, le DFB potentialisait l'effet des agonistes glutamate et quisqualate, en abaissant la valeur de leur CE50 (concentration d'agoniste nécessaire pour obtenir 50% de son effet maximal) d'un facteur supérieur à 2 (Tableau 1 et Figure 5). En présence d'une faible concentration d'agoniste, le DFB augmentait la production d'IP de façon "dose-dépendante" avec un CE50 de 1,81 ± 1,04 µM.

Le tableau 1 ci-dessous montre la potentialisation de l'effet du glutamate et du quisqualate sur mGlu5 par le DFB. Les valeurs de CE50 ont été déterminées à partir d'expériences d'effet dose du glutamate et du quisqualate en présence ou en absence de DFB, similaires à l'expérience décrite dans la figure 5. Les données correspondent à la moyenne ± écart type à la moyenne de (n) expériences.

**Tableau 1**

| | **Glutamate** | **Quisqualate** |
|---|---|---|
| **Base** | 47,6 ± 10,1 µM (5) | 20,3 ± 1,0 µM (1) |
| **100µM DFB** | 25,8 ± 5,7 µM (4) | 5,6 ± 1,3 µM (1) |

Sur les cellules exprimant la protéine mGlu5ΔS, le DFB stimulait la production d'IP, et se comportait donc comme un agoniste (Figure 6A). Cet effet du DFB dépendait de la concentration de DFB utilisée, avec une CE50 de 8,0 ± 3,0 µM (n=3), peu différente de la CE50 mesurée pour la potentialisation de l'effet du glutamate sur le récepteur sauvage, confirmant que le DFB agit au niveau du domaine heptahélice du récepteur. D'autre part, l'effet du DFB était inhibé par le MPEP, tant sur le récepteur sauvage (données non montrées) que sur la protéine mGlu5ΔS (Figure 6B), ce qui a confirmé que l'effet du DFB résultait de son action sur la protéine mGlu5ΔS.

En conséquence, le domaine heptahélice du récepteur mGlu5 (mGlu5ΔS) est seul capable d'activer les protéines G, dans la mesure où cette partie du récepteur mGlu5 est seule capable d'atteindre un état conformationnel actif. En outre, le régulateur allostérique négatif (antagoniste non-compétitif doué d'activité agoniste inverse) MPEP inhibe l'activité basale de cette protéine mGlu5ΔS, et le DFB est capable d'activer cette protéine. Aussi ces molécules n'ont-elles pas besoin du domaine externe pour agir.

### B. Autres récepteurs

Le protocole décrit dans la partie A.I. supra, concernant le récepteur mGlu5, a été appliqué *mutatis mutandis* aux récepteurs mGlu1, mGlu2 et à la sous-unité GB2.

Afin de démontrer que l'objet de la présente invention pour la recherche de modulateurs allostériques positifs s'applique à l'ensemble des RCPG-III, des récepteurs tronqués ont été générés selon la même approche que celle décrite pour la construction delta5s (mGlu5ΔS).

Pour cela, on a choisi des RCPG-III pour lesquels des modulateurs allostériques positifs ont été décrits: les récepteurs mGlu1 (Ro01-6128). (Knoflach et al., 2001), mGlu2 (LY487379) (Schaffhauser et al., 2003) et la sous-unité GABA_{B}2 (GB2) du récepteur GABA_{B} (CGP7930) (Urwyler et al., 2001). Les récepteurs tronqués de leurs parties extra et intracellulaires sont dénommés respectivement delta1s, delta2s et deltaGB2s et ont été exprimés de façon transitoire dans les cellules HEK293.

Afin de permettre aux récepteurs delta2s et deltaGB2s d'activer la phospholipase C, et donc d'augmenter la production d'inositolsphosphates, ces deux récepteurs ont été co-exprimés avec la protéine G chimère Gqi9 comme décrit précédemment (Gomeza et al., 1996 ; Franek et al., 1999).

Les composés Ro01-6128 et CGP7930 ont été synthétisés par F. Acher (Université René Descarte, Lab. de chimie et de biochimie pharmacologiques et toxicologiques, CNRS UMR-8601, 45, rue des Saints Pères, F-75270 Paris Cedex 06), et le composé LY487379 a été fourni par la société Addex Pharmaceuticals SA (12, Chemin Des Aulx, CH-1228, Plan Les Ouates, Genève, Suisse).
Comme décrit dans la littérature, les composés Ro01-6128 [Knoflach et al., 2001] et LY487379 [Schaffhauser et al., 2003] n'ont pas d'effet agoniste lorsqu'ils sont appliqués seuls sur les récepteurs mGlu1 et mGlu2, mais potentialisent l'effet des agonistes.
En revanche, comme illustré sur les figures 7 et 8, ces deux composés activent seuls les formes tronquées delta1s et delta2s.
De même, le CGP7930, qui n'a qu'une faible activité agoniste sur le récepteur hétérodimérique GABA_{B} (composé des sous-unités GABA_{B}1 ou GB1, et GABA_{B}2 ou GB2) (Urwyler et al., 2001), stimule fortement la construction tronquée deltaGB2s (Figure 9).

En définitive, les résultats décrits dans cette partie expérimentale démontrent que des protéines correspondant au domaine heptahélice des récepteurs RCPG-lll constituent des outils simples et efficaces pour identifier des régulateurs allostériques de ces récepteurs.

### REFERENCES

Bockaert J and Pin J-P (1999) Molecular tinkering of G-protein coupled receptors: an evolutionary success. EMBO J 18:1723-1729.
Brown EM and MacLeod RJ (2001) Extracellular calcium sensing and extracellular calcium signaling. Physiol Rev 81:239-297.
Conn P and Pin J-P (1997) Pharmacology and functions of metabotropic glutamate receptors: Ann Rev Pharmacol Toxicol 37:205-237.
Duthey B, Caudron S, Perroy J, Bettler B, Fagni L, Pin J-P and Prézeau L (2002) A single subunit (GB2) is required for G-protein activation by the heterodimeric GABAB receptor. J Biol Chem 277:3236-3241.
Joly C, Gomeza J, Brabet I, Curry K, Bockaert J and Pin J-P (1995) Molecular, functional and pharmacological characterization of the metabotropic glutamate receptor type 5 splice variants: comparison with mGlu1. J Neurosci 15:3970-3981.
Kaupmann K, Malitschek B, Schuler V, Heid J, Froestl W, Beck P, Mosbacher J, Bischoff S, Kulik A, Shigemoto R, Karschin A and Bettler B (1998) GABA B-receptor subtypes assemble into functional heteromeric complexes. Nature 396:683-687
Kniazeff J, Galvez T, Labesse G and Pin J-P (2002) No ligand binding in the GB2 subunit of the GABAB receptor is required for activation and allosteric interaction between the subunits. J Neurosci 22:7352-7361.
Marshall FH, Jones KA, Kaupmann K and Bettler B (1999) GABAB receptors - the first 7TM heterodimers. Trends Pharmacol Sci 20:396-9.
Nelson G, Chandrashekar J, Hoon MA, Feng L, Zhao G, Ryba NJ and Zuker CS (2002) An amino-acid taste receptor. Nature 416:199-202.
Pagano A, Rovelli G, Mosbacher J, Lohmann T, Duthey B, Stauffer D, Ristig D, Schuler V, Meigel I, Lampert C, Stein T, Prézeau L, Blahos J, Pin J-P, Froestl W, Kuhn R, Heid J, Kaupmann K and Bettler B (2001) C-terminal interaction is essential for surface trafficking but not for heteromeric assembly of GABA_{B} receptors. J Neurosci 21:1189-1202.
Pagano A, Rüegg D, Litschig S, Stoehr N, Stierlin C, Heinrich M, Floersheim P, Prézeau L, Carroll F, Pin J-P, Cambria A, Vranesic l, Flor PJ, Gasparini F and Kuhn R (2000) The non-compétitive antagonists 2-Methyl-6-(phenylethynyl)pyridine and 7-hydroxyiminocyclopropan[b]chromen-1a-carboxylic acid ethyl ester interact with overlapping binding pockets in the transmembrane region of group I metabotropic glutamate receptors. J Biol Chem 275:33750-33758.
Parmentier M-L, Prézeau L, Bockaert J and Pin J-P (2002) A model for the functioning of family 3 GPCRs. Trends Pharmacol Sci 23:268-274.
Pin J-P, Galvez T and Prezeau L (2003) Evolution, structure and activation mechanism of family 3/C G-protein coupled receptors. Pharmacol Ther in press.
Pollak MR, Brown EM, Chou Y-HW, Hebert SC, Marx SJ, Steinmann B, Levi T, Seidman CE and Seidman JG (1993) Mutations in the human Ca²⁺-sensing receptor gene cause familial hypocalciuric hypercalcemia and neonatal severe hyperparathyroidism. Cell 75:1297-1303.
Pollak MR, Brown EM, Estep HL, McLaine PN, Klfor O, Park J, Hebert SC, Seidman CE and Seidman JG (1994) Autosomal dominant hypocalcaemia caused by a Ca²⁺-sensing receptor gene mutation. Nature Genetics 8:303-307.
Williams DL, O'Brien JA, Lemaire W, Chen T-B, Chang RSL, Jacobson MA, Sur C, Pettibone DJ and Conn PJ (2002) DFB, an allosteric potentiator of mGlu5. Neuropharmacology 43:abs.
Ray and Northup (2002) Evidence for distinct cation and calcimimetic compound (NPS568) recognition domains in the transmembrane regions of the human Ca²⁺ receptor. J. Biol. Chem. 277: 18908-18913.
Hammerland L, Garrett J, Hung B., Levinthal C, Nemeth E (1998) Allosteric activation of the Ca2+ receptor expressed in Xenopus laevis oocytes by NPS 467 or NPS 568. Mol Pharmacol 53:1083-1088.
Hammerland LG, Krapcho KJ, Garrett JE, Alasti N, Hung BC, Simin RT, Levinthal C, Nemeth EF, Fuller FH (1999) Domains determining ligand specificity for Ca2+ receptors. Mol Pharmacol 55:642-648.
Nemeth E, Steffey M, Hammerland L, Hung B, Van Wagenen B, DelMar E, Balandrin M (1998) Calcimimetics with potent and selective activity on the parathyroid calcium receptor. Proc Natl Acad Sci (USA) 95:4040-4045.
Urwyler S, Mosbacher J, Lingenhoehl K, Heid J, Hofstetter K, Froestl W, Bettler B, Kaupmann K (2001) Positive allosteric modulation of native and recombinant GABAB receptors by 2,6-Di-tert.-butyl-4-(3-hydroxy-2,2-dimethyl-propyl)-phenol (CGP7930) and its aldehyde analogue CGP13501. Mol Pharmacol 60:963-971.
Knoflach F, Mutel V, Jolidon S, Kew JN, Malherbe P, Vieira E, Wichmann J, Kemp JA (2001) Positive allosteric modulators of metabotropic glutamate 1 receptor: Characterization, mechanism of action, and binding site. Proc Natl-Acad Sci U S A 98:13402-13407.
Kingston AE, Ornstein PL, Wright RA, Johnson BG, Mayne NG, Burnett JP, Belagaje R, Wu S, Schoepp. DD (1998) LY341495 is a nanomolar potent and selective antagonist of group II metabotropic glutamate receptors. Neuropharmacology 37:1-12.
Schoepp DD, Johnson BG, Wright RA, Salhoff CR, Mayne NG, Wu S, Cockerham SL, Burnett JP, Belegaje R, Bleakman D, Monn JA (1997) LY354740 is a potent and highly selective group II metabotropic glutamate receptor agonist in cells expressing human glutamate receptors. Neuropharmacology 36:1-11.
Berridge M (1983) Rapid accumulation of-inositol trisphosphate reveals that agonists hydrolyse polyphosphoinositides instead of phosphatidylinositol. Biochem J 212:849-858.
Brandish PE, Hill LA, Zheng W, Scolnick EM (2003) Scintillation proximity assay of inositol phosphates in cell extracts: High-throughput measurement of G-protein-coupled receptor activation. Anal Biochem 313:311-318.
Milligan G (2003) Principles: Extending the utility of [35S]GTPgS binding assays. Trends Pharmacol Sci 24:87-90.
Ango F, Albani-Torregrossa S, Joly C, Robbe D, Michel J-M, Pin J-P, Bockaert J, Fagni L (1999) A simple method to transfer plasmid DNA into neuronal primary cultures: functional expression of mGluR5 in cerebellar granule cells. Neuropharmacology 38:793-803.
Parmentier M-L, Pin J-P, Bockaert J, Grau Y (1996) Cloning and functional expression of a drosophila metabotropic glutamate receptor expressed in-the-embryonic central nervous system. J Neurosci 16:6687-6694.
Franek M, Pagano A, Kaupmann K, Bettler B, Pin J-P, Blahos Il J (1999) The heteromeric GABA-B receptor recognizes-G-protein α subunit C-termini. Neuropharmacology 38: 1657-1666.
Gomeza J, Mary S, Brabet I, Parmentier M-L, Restituito S, Bockaert J, Pin J-P (1996) Coupling of mGluR2 and mGluR4 to Gα15, Gα16 and chimeric Gaq/i proteins: characterization of new antagonists. Mol Pharmacol 50 : 923-930.
Schaffhauser HJ, Rowe BA, Morales S, Chavez-Noriega LE, Yin R, Jachec C, Rao SP, Bain G, Pinkerton AB, Vernier J-M, Bristow LJ, Varney MA, Daggett Lp (2003) Pharmacological characterization and identification of amino acids involved in the positive modulation of metabotropic glutamate receptor subtype 2. Mol Pharmacol 64: 798-810

## Revendications

1. Procédé d'identification et sélection d'un composé agissant comme régulateur allostérique d'un récepteur couplé aux protéines G de classe III (RCPG-III), comprenant au moins les étapes suivantes :
a) la production d'un RCPG-III recombinant (RCPG-III-Δ) essentiellement dépourvu du domaine extracellulaire du RCPG-III natif correspondant ;
b) la mise en présence dudit composé avec ledit RCPG-III-Δ ;
c) la mesure du niveau N₁ d'activité dudit RCPG-III-Δ en présence dudit composé ;
d) la comparaison du niveau N₁ mesuré à l'étape c) avec le niveau N₀ d'activité du RCPG-III-Δ en l'absence dudit composé ; et
e) si N₁ est significativement différent de N_{O}, l'identification et la sélection dudit régulateur allostérique,
**caractérisé en ce qu'**il est mis en oeuvre en l'absence d'agoniste du RCPG-III, **en ce que** ledit RCPG-III est choisi dans le groupe comprenant :
- les récepteurs activés par le glutamate ;
- les récepteurs activés par le GABA ;
- les récepteurs putatifs de phéromones ; et
- les récepteurs du goût ;
et **en ce que** ledit régulateur allostérique se comporte comme un agoniste ou un agoniste inverse dudit RCPG-III-Δ.

2. Procédé selon la revendication 1, **caractérisé en ce que** ledit RCPG-III-Δ est totalement dépourvu du domaine extracellulaire dudit RCPG-III natif.

3. Procédé selon la revendication 1, **caractérisé en ce que**, si N₁ est significativement supérieur à N₀, ledit régulateur allostérique est positif et se comporte comme un agoniste dudit RCPG-III-Δ.

4. Procédé selon la revendication 1, **caractérisé en ce que**, si N₁ est significativement inférieur à N₀, ledit régulateur allostérique est négatif et se comporte comme un agoniste inverse dudit RCPG-III-Δ.

5. Procédé selon la revendication 1 ou 2, **caractérisé en ce que** ledit RCPG-III-Δ comprend un domaine membranaire heptahélice.

6. Procédé selon la revendication 5, **caractérisé en ce que** ladite étape
a) comprend au moins les sous-étapes suivantes :
a1) l'amplification par PCR d'un acide nucléique codant ledit domaine membranaire dudit RCPG-III-Δ ;
a2) le clonage du produit d'amplification dans un vecteur d'expression recombinant ;
a3) la transfection dudit vecteur recombinant dans une cellule hôte recombinante ; et
a4) la production dudit RCPG-III-Δ par ladite cellule hôte recombinante.

7. Procédé selon la revendication 1 ou 2, **caractérisé en ce que** ledit RCPG-III-Δ comprend un signal d'adressage et un domaine membranaire heptahélice.

8. Procédé selon la revendication 7, **caractérisé en ce que** ledit signal d'adressage est un peptide signal.

9. Procédé selon la revendication 8, **caractérisé en ce que** ledit peptide signal est celui dudit RCPG-III natif correspondant.

10. Procédé selon l'une quelconque des revendications 7 à 9, **caractérisé en ce que** ladite étape a) comprend au moins les sous-étapes suivantes :
a1) l'amplification par PCR d'un acide nucléique codant ledit domaine membranaire dudit RCPG-III-Δ ;
a2) le clonage, dans un vecteur d'expression recombinant, du produit d'amplification en aval d'un acide nucléique codant ledit signal d'adressage ;
a3) la transfection dudit vecteur recombinant dans une cellule hôte recombinante ; et
a4) la production dudit RCPG-III-Δ par ladite cellule hôte recombinante.

11. Procédé selon la revendication 6 ou 10, **caractérisé en ce que** ladite cellule hôte recombinante est choisie parmi les cellules eucaryotes, telles que les cellules de levure et les cellules COS, et les cellules procaryotes, telles les cellules bactériennes.

12. Procédé selon l'une quelconque des revendications 1 à 11. **caractérisé en ce que** ledit RCPG-III est un récepteur activé par le glutamate.

13. Procédé selon la revendication 12, **caractérisé en ce que** ledit récepteur activé par le glutamate est choisi parmi les récepteurs mGlu1, mGlu2 et mGlu5.

14. Procédé selon l'une quelconque des revendications 1 à 11, **caractérisé en ce que** ledit RCPG-III est un récepteur activé par le GABA.

15. Procédé selon la revendication 14, **caractérisé en ce que** ledit récepteur activé par le GABA est la sous-unité GB2.

16. Application d'un procédé selon l'une quelconque des revendications 1 à 15, à l'identification et la sélection d'un composé utile à la mise en oeuvre d'un traitement prophylactique et/ou thérapeutique chez les mammifères, dont l'homme.

17. Application selon la revendication 16, **caractérisée en ce que** ledit traitement concerne au moins une maladie parmi : la maladie d'Alzheimer, la schizophrénie, les troubles nerveux, la dépression, l'épilepsie, la psychose, la dépendance aux drogues, la douleur.

18. Application d'un procédé selon l'une quelconque des revendications 1 à 15, à l'identification et la sélection d'un composé utile à la mise en oeuvre d'un traitement destiné à augmenter ou réduire la prolifération d'organismes vivants, tels les insectes et les vers.

19. Application d'un procédé selon l'une quelconque des revendications 1 à 11, à l'identification et la sélection d'un composé utile comme additif alimentaire.

20. Application selon la revendication 19, **caractérisée en ce que** ledit additif alimentaire confère un goût sucré, tel un édulcorant, ou un goût umami, ou augmente la sensibilité de détection d'un composé sucré.

## Claims

1. Method for identifying and selecting a compound that acts as an allosteric regulator of a class III G protein-coupled receptor (GPCR-III), comprising at least the following steps:
a) producing a recombinant GPCR-III (GPCR-III-Δ) essentially depleted of the extracellular domain of the corresponding native GPCR-III;
b) bringing said compound into contact with said GPCR-III-Δ;
c) measuring the level N₁ of activity of said GPCR-III-Δ in the presence of said compound;
d) comparing the level N₁ measured in step c) with the level N₀ of activity of the GPCR-III-Δ in the absence of said compound; and
e) if N₁ is significantly different from N₀, identifying and selecting said allosteric regulator,
**characterized in that** it is implemented in the absence of an agonist of GPCR-III, **in that** said GPCR-III is chosen from the group comprising:
- glutamate-activated receptors;
- GABA-activated receptors ;
- putative pheromone receptors; and
- taste receptors,
and **in that** said allosteric regulator behaves like an agonist or an inverse agonist of said GPCR-III-Δ.

2. Method according to Claim 1, **characterized in that** said GPCR-III-Δ is completely depleted of the extracellular domain of said native GPCR-III.

3. Method according to Claim 1, **characterized in that**, if N₁ is significantly greater than N₀, said allosteric regulator is positive and behaves like an agonist of said GPCR-III-Δ.

4. Method according to Claim 1, **characterized in that**, if N₁ is significantly less than N₀, said allosteric regulator is negative and behaves like an inverse agonist of said GPCR-III-Δ.

5. Method according to Claim 1 or 2, **characterized in that** said GPCR-III-Δ comprises a heptahelical membrane domain.

6. Method according to Claim 5, **characterized in that** said step a) comprises at least the following substeps:
a1) PCR amplification of a nucleic acid encoding said membrane domain of said GPCR-III-Δ;
a2) cloning of the amplification product into a recombinant expression vector;
a3) transfection of said recombinant vector into a recombinant host cell; and
a4) production of said GPCR-III-Δ by said recombinant host cell.

7. Method according to Claim 1 or 2, **characterized in that** said GPCR-III-Δ comprises a targeting signal and a heptahelical membrane domain.

8. Method according to Claim 7, **characterized in that** said targeting signal is a signal peptide.

9. Method according to Claim 8, **characterized in that** said signal peptide is that of said corresponding native GPCR-III.

10. Method according to any one of Claims 7 to 9, **characterized in that** said step a) comprises at least the following substeps:
a1) PCR amplification of a nucleic acid encoding said membrane domain of said GPCR-III-Δ;
a2) cloning, into a recombinant expression vector, of the amplification product downstream of a nucleic acid encoding said targeting signal;
a3) transfection of said recombinant vector into a recombinant host cell; and
a4) production of said GPCR-III-Δ by said recombinant host cell.

11. Method according to Claim 6 or 10, **characterized in that** said recombinant host cell is chosen from eukaryotic cells, such as yeast cells and COS cells, and prokaryotic cells such as bacterial cells.

12. Method according to any one of Claims 1 to 11, **characterized in that** said GPCR-III is a glutamate-activated receptor.

13. Method according to Claim 12, **characterized in that** said glutamate-activated receptor is chosen from mGlu1, mGlu2 and mGlu5 receptors.

14. Method according to any one of Claims 1 to 11, **characterized in that** said GPCR-III is a GABA-activated receptor.

15. Method according to Claim 14, **characterized in that** said GABA-activated receptor is the GB2 subunit.

16. Application of a method according to any one of Claims 1 to 15, for identifying and selecting a compound that is useful for carrying out a prophylactic and/or therapeutic treatment in mammals, including humans.

17. Application according to Claim 16, **characterized in that** said treatment concerns at least one disease among: Alzheimer's disease, schizophrenia, nervous disorders, depression, epilepsy, psychosis, drug dependency and pain.

18. Application of a method according to any one of Claims 1 to 15, for identifying and selecting a compound that is useful for carrying out a treatment intended to increase or reduce the proliferation of living organisms, such as insects and worms.

19. Application of a method according to any one of Claims 1 to 11, for identifying and selecting a compound that is useful as a food additive.

20. Application according to Claim 19, **characterized in that** said food additive confers a sweet taste, such as a sweetener, or an umami taste, or increases the sensitivity of detection of a sweet compound.

## Patentansprüche

1. Verfahren zur Identifikation und Auswahl einer Verbindung, die als allosterischer Regulator eines Rezeptors wirkt, der an G-Proteine der Klasse III gekoppelt ist (GPCR-III), umfassend mindestens die folgenden Schritte:
a) die Produktion eines rekombinanten GPCR-III (GPCR-III-Δ), dem im Wesentlichen die extrazelluläre Domäne des entsprechenden nativen GPCR-III fehlt;
b) das In-Kontakt-Bringen der Verbindung mit dem GPCR-III-Δ;
c) das Messen des Aktivitätsniveaus N₁ des GPCR-III-Δ in Anwesenheit der Verbindung;
d) den Vergleich des Niveaus N₁, das im Schritt c) gemessen wurde, mit dem Aktivitätsniveau N₀ des Rezeptors GPCR-III-Δ in Abwesenheit der Verbindung; und,
e) wenn N₁ von N₀ signifikant verschieden ist, die Identifikation und Auswahl des allosterischen Regulators,
**dadurch gekennzeichnet, dass** es in Abwesenheit von Agonisten des GPCR-III durchgeführt wird, und **dadurch**, dass der GPCR-III ausgewählt ist aus der Gruppe, welche umfasst:
- durch Glutamat aktivierte Rezeptoren;
- durch GABA aktivierte Rezeptoren;
- mutmaßliche Rezeptoren von Pheromonen; und
- Geschmacksrezeptoren;
und **dadurch**, dass der allosterische Regulator sich wie ein Agonist oder ein inverser Agonist des GPCR-III-Δ verhält.

2. Verfahren nach Anspruch 1, **dadurch gekennzeichnet, dass** dem GPCR-III-Δ die extrazelluläre Domäne des nativen GPCR-III vollständig fehlt.

3. Verfahren nach Anspruch 1, **dadurch gekennzeichnet, dass**, wenn N₁ signifikant über N₀ liegt, der allosterische Regulator positiv ist und sich wie ein Agonist des GPCR-III-Δ verhält.

4. Verfahren nach Anspruch 1, **dadurch gekennzeichnet, dass**, wenn N₁ signifikant unter N₀ liegt, der allosterische Regulator negativ ist und sich wie ein inverser Agonist des GPCR-III-Δ verhält.

5. Verfahren nach Anspruch 1 oder 2, **dadurch gekennzeichnet, dass** der GPCR-III-Δ eine Heptahelix-Membrandomäne umfasst.

6. Verfahren nach Anspruch 5, **dadurch gekennzeichnet, dass** der Schritt a) mindestens die folgenden Unterschritte umfasst:
a1) Amplifikation einer Nukleinsäure, welche die Membrandomäne des GPCR-III-Δ kodiert, mittels PCR;
a2) Klonieren des Ämplifikationsproduktes in einen rekombinanten Expressionsvektor;
a3) Transfektion des rekombinanten Vektors in eine rekombinante Wirtszelle; und
a4) Produktion des GPCR-III-Δ durch die rekombinante Wirtszelle.

7. Verfahren nach Anspruch 1 oder 2, **dadurch gekennzeichnet, dass** der GPCR-III-Δ ein Adressierungssignal und eine Heptahelix-Membrandomäne umfasst.

8. Verfahren nach Anspruch 7, **dadurch gekennzeichnet, dass** das Adressierungssignal ein Peptidsignal ist.

9. Verfahren nach Anspruch 8, **dadurch gekennzeichnet, dass** das Peptidsignal jenes des entsprechenden nativen GPCR-III ist.

10. Verfahren nach irgendeinem der Ansprüche 7 bis 9, **dadurch gekennzeichnet, dass** der Schritt a) mindestens die folgenden Unterschritte umfasst:
a1) Amplifikation einer Nukleinsäure, welche die Membrandomäne des GPCR-III-Δ kodiert, mittels PCR;
a2) Klonieren des Amplifikationsprodukts in einen rekombinanten Expressionsvektor stromabwärts von einer Nukleinsäure, die das Adressierungssignal kodiert;
a3) Transfektion des rekombinanten Vektors in eine rekombinante Wirtszelle; und
a4) Produktion des GPCR-III-Δ durch die rekombinante Wirtszelle.

11. Verfahren nach Anspruch 6 oder 10, **dadurch gekennzeichnet, dass** die rekombinante Wirtszelle ausgewählt ist aus eukaryotischen Zellen, wie Hefezellen und COS-Zellen, und prokaryotischen Zellen, wie Bakterienzellen.

12. Verfahren nach irgendeinem der Ansprüche 1 bis 11, **dadurch gekennzeichnet, dass** der GPCR-III ein durch Glutamat aktivierter Rezeptor ist.

13. Verfahren nach Anspruch 12, **dadurch gekennzeichnet, dass** der durch Glutamat aktivierte Rezeptor ausgewählt ist aus den Rezeptoren mGlu1, mGlu2 und mGlu5.

14. Verfahren nach irgendeinem der Ansprüche 1 bis 11, **dadurch gekennzeichnet, dass** der GPCR-III ein durch GABA aktivierter Rezeptor ist.

15. Verfahren nach Anspruch 14, **dadurch gekennzeichnet, dass** der durch GABA aktivierte Rezeptor die Untereinheit GB2 ist.

16. Anwendung eines Verfahrens nach irgendeinem der Ansprüche 1 bis 15 zur Identifikation und Auswahl einer Verbindung, die zur Durchführung einer prophylaktischen und/oder therapeutischen Behandlung bei Säugern, darunter dem Menschen, nützlich ist.

17. Anwendung nach Anspruch 16, **dadurch gekennzeichnet, dass** die Behandlung mindestens eine Krankheit unter den folgenden betrifft: Alzheimer-Krankheit, Schizophrenie, Nervenstörungen, Depression, Epilepsie, Psychose, Drogenabhängigkeit, Schmerzen.

18. Anwendung eines Verfahrens nach irgendeinem der Ansprüche 1 bis 15 zur Identifikation und Auswahl einer Verbindung, die zur Durchführung einer Behandlungen nützlich ist, die dazu bestimmt ist, die Proliferation von lebenden Organismen, wie Insekten und Würmern/Maden/Raupen, zu erhöhen oder zu verringern.

19. Anwendung eines Verfahrens nach irgendeinem der Ansprüche 1 bis 11 zur Identifikation und Auswahl einer Verbindung, die als Nahrungsmittelzusatz nützlich ist.

20. Anwendung nach Anspruch 19, **dadurch gekennzeichnet, dass** der Nahrungsmittelzusatz einen Zuckergeschmack wie ein Süßmittel oder einen Umami-Geschmack verleiht oder die Nachweisempfindlichkeit einer Zuckerverbindung erhöht.
